# EUROPEAN PATENT APPLICATION

(11) **EP 1 002 803 A1**
(43) Date of publication of application: **24.05.2000**
(21) Application number: 98203779.8
(22) Date of filing: 10.11.1998
(51) Int. Cl.: C07K 14/42, A61K 38/16, G01N 33/68

(54) **Method for the isolation of legume lectins and their use as a medicament**

(71) Applicant: K.U. Leuven Research & Development, 3000 Leuven (BE); Peumans, Willy Gerard Jean Ghislain, 3201 Langdorp-Aarschot (BE); VanDamme, Els Jeanine Maria, 3201 Langdorp-Aarschot (BE)
(72) Inventor: Peumans, Willy Gerard Jean Chislain, 3201 Langdors-Anrshot (BE)
(74) Representative: Hoorweg, Petrus Nicolaas

(57) **Abstract**

A method for the isolation of legume lectins, in particular one or more of the tetramers a₄, a₃b₁, a₂b₂, a₃b₁ and b₄ comprising the following steps:
(i) an extraction of legume lectins from raw material,
(ii) a preparation of a partially purified albumin fraction by selective precipitation of non-lectin proteins and polysaccharides
(iii) a purification comprising a chromatography step, and
   purified legume lectins, in particular the isoforms a₄ and/or b₄, obtained in accordance with said method for use as a medicament.

## Description

### Field of the invention

The present invention relates to a novel method for the isolation of legume lectins in particular one or more a₄, a₃b₁, a₂b₂, a₁b₃, b₄ isolectins and to the obtained purified for their use as a medicament.

Lectins are carbohydrate-binding proteins and commonly found in plants of the Leguminoseae (Fabaceae) family , for example in beans (Phaseolus vulgaris). In particular the tree Robinia pseudoacacia (or black locust) is known to contain legume lectins in bark and seeds.

### Description of the prior art

Detailed biochemical and molecular biological studies have demonstrated that the seed and bark lectins are encoded by two different sets of genes. At present the following lectins and lectin genes have been described:
Seeds contain two different homotetrameric lectins:
   RPsAI: a homotetramer of four identical glycosylated subunits of 34 kDa, which are encoded by LECRPAs1.
   RPsAII: a homotetramer of four identical glycosylated subunits of 29 kDa, which are encoded by LECRPAs2.
Bark contains a complex mixture of lectins:
   RPbAI: this major lectin is composed of five isolectins that originate from the association of a-subunits (32 kDa) and b-subunits (29 kDa) into homo- and hetero-tetramers. According to their subunit composition the isolectins are designated as isoforms a₄, a₃b₁, a₂b₂, a₁b₃ and b₄, respectively, The a- and b-subunits are encoded by the lectin genes LECRPA1 and LECRPA2, respectively.
   RPAbII: this minor lectin is a homotetramer of c-subunits (26 kDa), which are encoded by LECRPA3.

This invention is in particular, but not exclusive, directed to a method for the isolation the lectins of the black locust, and more in particular to the RPbAI tetramers, i.e. a₄, a₃b₁, a₂b₂, a₁b₃ and b₄. In a prefered embodiment the homotetramers a₄ and b₄ are isolated.

All black locust lectins belong to the legume lectin family. Since none of the lectins is inhibited by any simple sugar at reasonable concentrations it is generally accepted that the Robinia pseudoacacia lectins possess a complex binding site which accommodates preferentially complex glycans containing galactose and/or N-acetyl-galactosamine residues. By virtue of this particular specificity the black locust lectins recognize and bind typical complex N-glycans from both animal and human origin, and accordingly are able to interact with human and animal cells. It has been previously demonstrated, for example, that both the seed (Wantyghem et al., 1986) and bark (Horejsi et al., 1978) lectins are mitogenic for human peripheral-blood lymphocytes.

Due to their unique biological activities towards human and animal cells, the Robinia pseudoacacia lectins, and especially RPsAI and RPbAI are prefered candidates for some particular biomedical and/or therapeutical applications. Since such applications require large quantities of highly purified protein, the availability of sufficient amounts of pure lectin or isolectin will be a prerequisite for further exploitation of the biological and therapeutical properties of any lectin, and in particular of the black locust lectin.

In the past, most of the research on the Robinia pseudoacacia bark lectins was done with complex mixtures of isolectins. As a matter of fact, the unraveling of the mechanism underlying the complex isolectin composition of the black locust bark has been achieved only recently (Van Damme et al., 1995) and was based for a great deal on the first successful resolution of the bark isolectins on an analytical scale. However, this resolution required a complex purification and separation scheme including affinity chromatography and analytical ion-exchange chromatography and yielded only very small quantities (< 1 mg) of pure isolectins. Since this method can not be scaled up to a preparative and a fortiori not to an industrial scale, it is an object of the invention to provide a novel method simplifying the purification of large quantities of lectins.

To highlight the advantages of our novel method the existing methods to purify the Robinia pseudoacacia bark lectins and isolectins are given hereunder.

A first method to isolate the Robinia pseudoacacia bark lectins has been described in 1978 by Horejsi et al. (Horejsi et al., 1978). This method included:
- stripping of the bark
- drying of the bark
- milling of the dry bark
- extraction of the lectin by stirring the powdered bark in saline (i.e. 0.9% NaCl)
- clearing of the crude extract by centrifugation
- ammonium sulphate precipitation of the proteins (700 g ammonium sulphate per liter extract)
- collection of the precipitated proteins by centrifugation
- dialysis
- lyophilization of the dialysate
- dissolution of the lyophilizate in saline
- addition of formalinized human erythrocytes
- adsorption of the lectins to formalinized human erythrocytes
- six washes of the formalinized human erythrocytes with saline
- desorption of the lectins from the formalinized human erythrocytes with three subsequent washes of the cells with 10% Na-tetraborate (pH 9.0)
- dialysis of the desorbed lectin against water
- concentration by pervaporation in a dialysis tube
- clearing by centrifugation
- lyophilization of the supernatant
   Final yield: 350 mg total lectin (i.e. a mixture of RPbAI isoforms) from 300 g of bark.

This method is very complex and time consuming because it includes an ammonium sulphate precipitation and several dialysis steps. In addition, the adsorption on formalinized human erythrocytes causes a serious problem because the final lectin preparations may be contaminated with formaldehyde (which precludes any therapeutic application of the lectin). An other disadvantage is that this method does not allow to separate the different isoforms of RPbAI.

A second known method has been described by Tazaki and Yoshida (Takazaki and Yoshida, 1992). This method included:
- stripping of the bark
- freezing of the bark in liquid nitrogen
- pulverization of the frozen bark in a blender
- extraction of the lectin in PBS: phosphate buffered saline (1.5 mM KH₂PO₄, 10 mM Na₂HPO₄, 3 mM KCl, 140 mM NaCl, pH 7.4) using a Polytron homogenizer
- clearing of the extract by centrifugation
- dialysis of the supernatant
- chromatography on fetuin-agarose
- ammonium sulphate precipitation (80% saturation) of the unbound proteins (including the lectin)
- collection of the precipitated proteins by centrifugation
- dialysis
- ion exchange chromatography on DEAE-Sepharose CL-6B
- elution of the lectin with a gradient of increasing salt concentration
- pooling and dialysis of the lectin containing fractions
- centrifugation
- ion exchange chromatography of the supernatant on CM-Toyopearl
- elution of the lectin with a gradient of increasing salt concentration
- pooling of the active fractions
- dialysis against PBS: phosphate buffered saline (1.5 mM KH₂PO₄, 10 mM Na₂HPO₄, 3 mM KCl, 140 mM NaCl, pH 7.4)
   Final yield: 5 mg lectin from 100 g of bark. The lectin was apparently strongly enriched in isolectin b₄ of RPbAI.
This method also is very complex and time consuming because it includes freezing in liquid nitrogen, an ammonium sulphate precipitation and several dialysis steps. In addition, the overall yield is low and the final lectin preparation contains little if any RPbAI isolectin a₄.

A third method has been described by Van Damme et al. (Van Damme et al., 1995). This method included:
- stripping of the bark
- freezing of the bark at -20°C
- homogenization of the frozen bark in 20 mM acetic acid using a Waring blender
- filtering of the homogenate through cheese cloth and adjustment of the pH to 5.0 with 1 N NaOH
- clearing of the extract by centrifugation
- addition of 1.5 g/l CaCl₂ followed by adjustment of the pH to 9.0 with 1 N NaOH
- clearing of the extract by centrifugation
- lowering of the pH to 3.8 with 1 N HCl
- clearing of the extract by centrifugation
- adjustment of the pH to 7.0 with 1 N NaOH
- addition of ammonium sulphate (final concentration 1 M)
- degassing and centrifugation
- affinity chromatography on fetuin-agarose in the presence of 1 M ammonium sulphate
- elution of the lectin with 20 mM unbuffered 1,3 diaminopropane
- neutralization of the desorbed fraction with 1N HCl
- addition of ammonium sulphate (final concentration 1 M)
- centrifugation
- adsorption of supernatant on phenyl-Sepharose
- elution of the lectin with a gradient of decreasing ammonium sulphate concentration
- pooling of lectin-containing fractions
- dialysis against Na-formate-buffer
- ion exchange chromatography on an analytical Mono-S column
- manual collection of the individual isolectin peaks
   Final yield: a few hundred µg of each isolectin of RPbAI.

This is the only method described heretofore to separate the individual RPbAI isolectins. Although the method allows to obtain analytical quantities of the pure isolectins it is not suited for the preparation of large quantities of lectins because the complex procedure is only applicable to small amounts of starting material and in addition is very time consuming.

In conclusion, no document is known providing a suitable method for the isolation of large quantities and purified lectins.

### Description of the invention

The novel method of the present invention comprises the following steps:
(i) an extraction of legume lectins from raw material,
(ii) a preparation of a partially purified albumin fraction by selective precipitation of non-lectin proteins and polysaccharides, and
(iii) a purification of lectins, whereby the purification comprises a chromatography step. Due to an adequate selective precipitation step (ii) the fractions of the legume lectins obtained in step (iii) are highly purified and concentrated.

This inventive feature resides in the fact that a spontaneous crystallisation occurs of the isoforms a₄ and b₄ of lectin. A prefered embodiment of the invention comprises the purification of the fractions of a₄ and b₄ obtained in (iii) are crystallized upon standing in a cold environment, preferably from 0-10°C.

A further purification of the a₄ and b₄ is performed inobtainably by a recrystallization, preceded by an additional chromatography step.

In a prefered embodiment of the invention the extaction step is performed in the presence of an ascorbic acid solution used as a buffer and anti-oxidantia. Ascorbic acid (vitamin C) is in minor concentration harmless or even positive for humans and animals.

To further optimize the extraction in terms of both yield and lectin activity the following steps are in combination or seperately implemented in prefered embodiments of the invention.

Regarding to the raw material several important precautions could be made with respect to the choice and the preparation. As raw material the bark of Robinia pseudoacacia is prefered due to the sufficient high level of lectins.

In the choice of the bark of the black locust several other prefered features could be taken into consideration.
a) The lectin content of black locust bark is developmentally regulated and subjected to strong seasonal changes. It is prefered, therefore, to collect the raw materials in a period coinciding with a maximal lectin content. In temperate regions black locust is a deciduous tree. Lectin levels are maximal in the period between the shedding of the leaves in fall and the formation of new sprouts in springtime. Due to physiological changes occurring during early spring the moisture content of the bark increases. Concomitantly, insoluble reserve polysaccharides are progressively converted into soluble poly- and oligosaccharides, which seriously hamper the purification of the proteins. Therefore, bark should preferably be collected before these physiological changes take place.
b) Lectins are located almost exclusively in the living (i.e. inner) tissue of the bark. The outer dead tissue consists mainly of corky material and contains a lot of highly reactive phenolic compounds which may react with and partially inactivate biologically active proteins such as RPA. The thickness of the outer layer of dead material increases with age. It is very important to reduce contamination with dead tissue as much as possible. Therefore, the corky layer is removed when the bark is stripped from the trees.
c) Fresh bark contains up to 50% water and is susceptible to microbial decay. To prevent spoilage the fresh bark is preferably stored frozen or lyophilized.

Further prefered embodiments of the extraction step of the invention comprises combined or separately substeps:
a) Milling of fractionizing of the lyophilized bark.
b) Extraction by stirring in a solution of ascorbic acid.
c) Selective extraction of the lectin. The pH of the extract is adjusted between 2 and 4, preferably between 2,5 and 3,5. Under these conditions the lectins remain in solution whereas most other proteins precipitate upon standing overnight in the cold.

The preparation of a partially purified albumin fraction has the effect of a selective precipitation of non-lectin proteins and polysaccharides.

Preferably of the selective precipitation comprises combined or separately:
centrifugation of the obtained extract, subsequently a decantation of the supernatant, whereby a salt is added, preferably CaCl₂ and whereby the pH is raised between 8 and 10,5 and preferably around 9, such that any co-extracted polysaccarides precipitate, and
further a decantation of the obtained supernatant.

A further advantage of the method of the invention is that no high speed centrifugation is required.

A further prefered embodiment of the method of the invention is that the obtained supernatant in step (ii) is being purified by ion exchange chromatography, whereby the resulting concentration of the obtained fraction of the isoforms a₄, b₃a, b₂a₂, b₁a₃ and/or b₄ is at least 5 mg/ml and preferably 10 mg/ml or more.

The purification step is achieved by ion exchange or affinity chromatography and crystallization. During the chromatography step advantage is taken of the fact that the affinity of the RPbAI isolectins for the ion exchanger increase with their b-subunit content (this means that the affinity increases from a₄ over a₃b₁,, a₂b₂, and a₁b₃ to b₄). Accordingly, the different isolectins elute in the order a₄, a₃b₁, a₂b₂, a₁b₃ and b₄ upon increasing the pH or ionic strength of the running buffer.

After the chromatografy step the eluate is suitably pure and enriched that crystals are formed, in particular a₄ and b₄ crystals.

Hereunder the method of the invention is elucidated in a prefered embodiment for the isolation of the isolectin a₄.

The lectin isolation procedure, which basically can be subdivided in (i) extraction, (ii) preparation of a partially purified albumin fraction and (iii) purification of legume lectins includes in the most prefered embodiment the following steps:
(i) Extraction: to optimize the extraction in terms of both yield and lectin activity the following steps are included :
   a) The lyophilized bark is fractionated, for example milled. The resulting meal can be extracted in an aqueous medium without vigorous agitation or mixing. In this way oxidation by air oxygen is minimized.
   b) The meal is extracted by gentle stirring for 1 h at 0-5°C in a solution of 0.1% ascorbic acid. Ten liters are used per kg of bark meal.
   c) Selective extraction of the lectin. The pH of the extract is adjusted to 3.0 with 1 N HCl. Under these conditions RPbAI remains in solution whereas most other proteins precipitate upon standing overnight in the cold.
(ii) Preparation of a partially purified albumin fraction by selective precipitation of non-lectin proteins and polysaccharides.
   a) The crude extract is centrifuged at low speed (3,000 g)
   b) The supernatant is decanted, CaCl₂ is added to a final concentration of 1 g/l and the pH raised to 9.5 by the addition of 1N NaOH. Under these conditions the bulk of extracted polysaccharides precipitate upon standing in the cold overnight.
   c) The supernatant is carefully decanted. No centrifugation is required except for the settled precipitate (about 5% of the total volume) which can be clarified by centrifugation at low speed (3,000 g). The procedure followed from (ii)a to (ii)c yields an extract highly enriched in RPbAI and does not require a high speed centrifugation.
(iii) Purification. The purification of isolectin, in particular a₄ and b₄ is achieved by ion exchange chromatography and crystallization. During the ion exchange chromatography step advantage is taken of the fact that the affinity of the RPbAI isolectins for the ion exchanger increase with their b-subunit content (this means that the affinity increases from a₄ over a₃b₁, a₂b₂, and a₁b₃ to b₄). Accordingly, the different isolectins elute in the order a₄, a₃b₁ , a₂b₂, a₁b₃ and b₄) upon increasing the pH or ionic strength of the running buffer.
   Due to the small difference in isoelectric point between the a- and b-subunits of RPbAI the different isoforms cannot be completely resolved by ion exchange chromatography on a preparative scale. It is possible, however, to obtain a concentrated preparation of RPbAI which is sufficiently enriched in each isoform. The isoform a₄ and b₄ are suitable for further purification by selective crystallization of isoform a₄ and b₄. Hereunder the purification is as a prefered embodiment of the invention elaborated for the isoform a₄.
   a) The partially purified albumin fraction obtained sub (ii)c is adjusted to pH 2.8 with 1 N HCl and diluted with an equal volume of distilled water.
   b) The diluted extract is loaded onto a column of a sulphopropyl-based cation exchanger (e.g. SFF from Pharmacia) at pH 2.8. To ensure complete binding of RPbAI, 200 ml settled gel is required for 10 l of this diluted extract. At the end of the loading of the column, binding of the lectin is checked by agglutination assays with trypsin-treated human red blood cells (type A). Ten µl of the eluate is mixed with 10 µl of 0.1M Tris-HCl (pH 7.8), 80 µl of a 1% suspension of erythrocytes is added and the agglutination checked visually after 5 minutes. A parallel test with the complete (i.e. before loading on the ion exchange column) extract is included as a positive control of the agglutination test.
   c) After washing of unbound proteins the column is eluted with 0.1 M Na-acetate pH 5.0. The eluate consists almost exclusively of RPbAI and is considerably enriched in RPbAI isolectin a₄.
   d) The eluates of different runs sub (iii)c are pooled, brought at pH 2.8 with 1 N HCl and reloaded on a column of a sulphopropyl-based cation exchanger at pH 2.8. About 50 mg protein are loaded per ml of settled gel in order to maximally exploit the capacity of the column. After loading the proteins, the column is equilibrated with 5 volumes of 0.1 M Na-acetate pH 4.5 and subsequently eluted with a linear gradient of increasing NaCl concentration (from 0 to 0.2 M in 0.1 M Na-acetate pH 4.5). The total volume of the gradient corresponds to 5 volumes of settled gel and the A₂₈₀ of the eluate is continuously monitored. As soon as the A₂₈₀ of the effluent surpasses 2.0 fractions of 25 ml are collected in 50 ml plastic Falcon tubes.
   e) Fractions are stored at 0-2°C for 5 days. During storage, RPbAI isolectin a₄- crystallizes. This crystallization process occurs only above a threshold concentration (about 5 and prefered about 10 mg/ml) of RPbAI isolectin a₄. It is important therefore that the total amount of lectin loaded on the column is sufficiently high to yield fractions with a high concentration of RPbAI isolectin a₄.
   f) Crystals formed upon storage in the cold are dissolved in 50 mM Tris-HCl pH 8.7 and the lectin solution is centrifuged (9,000 g for 15 min) to precipitate the undissolved material. The supernatant is adjusted to pH 2.8 with 1 N HCl and rechromatographed as described under point (iii)d-e. RPA isolectin a₄ is allowed to crystallize for 5 days.
   g) Crystals are removed by gentle centrifugation, dissolved in 50 mM Tris-HCl pH 8.7, dialyzed against distilled water and lyophilized. This preparation contains highly pure RPbAI isolectin a₄.
   h) The lyophilized RPbAI isolectin a₄ is stored at -20°C until use.
   To increase the overall yield of RPbAI isolectin a₄ the supernatants of the crystal-containing fractions (sub (iii)e and (iii)g) of different runs are pooled and rechromatographed as described under (iii)d-g.

### Overall yield

Using the above described procedure about 1 g RPA isolectin a₄ is obtained per kg of fresh bark tissue.

As an alternative to ion exchange chromatography a purification scheme for pure RPbAI isoforms based on affinity chromatography on different affinity supports (including galactose-Sepharose 4B, N-acetylgalactosamine-Sepharose 4B, fetuin-Sepharose 4B, asialofetuin-Sepharose 4B, mucin-Sepharose 4B and asialomucin-Sepharose 4B) is described hereunder. RPbAI was partly retained on some affinity supports and in addition eluted upon washing the column with PBS: phosphate buffered saline (1.5 mM KH₂PO₄, 10 mM Na₂HPO₄, 3 mM KCl, 140 mM NaCl, pH 7.4). As a result the yield was low (less than 5 % of the total lectin was retained). The overall yield of the affinity chromatography could stronly be increased (up to near 100 % recovery of RPbAI) by doing the chromatography in 2 M ammonium sulphate (adjusted to pH 7.5) followed by desorption of the lectin with water.

To enhance the efficiency of ion exchange chromatographic techniques a method was developed to get rid of as much as possible contaminating proteins and other interfering biopolymers during the extraction process. A partially purified albumin fraction highly enriched in RPbAI (90-95 % of the total protein) could be obtained through a selective extraction of the lectin at low pH (i.e. in a 0.1 % solution of using ascorbic acid), followed by a precipitation of the insoluble proteins at pH 3.0, and a precipitation of pectic substances at pH 9.0 in the presence of 1g/l CaCl₂.

This partially purified albumin fraction exhibited better flow properties upon ion exchange chromatography and yielded a RPbAI preparation with a slightly improved purity (less than 5 % contaminating proteins). However, pure RPbAI isoform [a]₄ and [b]₄ could still not be obtained because the different isoforms of RPbAI could not be completely resolved by this technique.
(iii) The problem of the isolation of pure RPbAI isoform [a]₄ and [b]₄ could eventually be resolved by the introduction of a crystallization step. Partially purified RPbAI isoforms [a]₄ and [b]₄, which could be obtained by ion exchange chromatography, were subjected to a second ion exchange chromatography step. Elution of these partially purified RPbAI isoforms [a]₄ and [b]₄ by a gradient of increasing NaCl concentration yielded fractions from which isoforms [a]₄ and [b]₄ spontaneously crystallized upon standing in the cold. After completion of the crystallization process (taking 5-10 days) the crystallized proteins can be recovered yielding preparations of virtually pure RPbAI isoforms [a]₄ and [b]₄. Complete purity was eventually achieved by a recrystallization step (preceded by an additional ion exchange chromatography of the redissolved crystals).

Taken into account that the seed lectins are minor proteins whereas the bark lectins are predominant proteins, any commercial exploitation of the black locust lectin(s) will be based preferentially on bark proteins. In addition, considering the fact that there is a substantial difference in biological activities between the different isoforms of the bark lectins, there will be a need for large quantities of the most active isoform in casu RPbAI isolectin a₄ and b₄.

Interestingly, RPsAI and RPbAI were about one order of magnitude more active than RPsAII and RPbAII, respectively.

The novel and powerful method according to the invention has in particular been developed to isolate large quantities of isoform a₄ and b₄. Our procedure is far superior to all previously described methods to isolate and purify black locust bark lectins and isolectins and can easily be scaled up to any desired production level.

The novel method of the invention differs from the previously described methods in:
- the optimization of the choice and preparation of the raw materials
- the introduction of a partial purification based on selective extraction of lectins and selective removal of contaminants through the use of very specific conditions
- the introduction of a double ion exchange chromatography step using different conditions for a single ion exchange matrix
- the introduction of a unique highly specific crystallization step
- a further recrystallization step

The novel method offers several important advantages in comparison to the previously described methods
- due to the selective extraction procedure centrifugation is limited to a minimum and can be done at relatively low speed
- no ammonium sulphate precipitation step is included
- no affinity matrix is required
- all chromatographic separations can be done on a single column
- the process can easily be automated
- large quantities of raw materials can easily be handled
- the isolation procedure requires only minimal quantities of chemicals, which allows to reduce the production of noxious or dangerous chemical waste to an absolute minimum
- the final product is obtained in a very concentrated form

The invention also relates to the use of the isolated isoforms as a medicament, in particular the isoforms a₄ and b₄, which are useful as a mitogen and more in particular in the treatment of cancer.

### Therapeutical and diagnostic applications of pure isoforms of legume lectins

### (i) Isoform [a₄] of RPbAI

RPbAI isoform [a]₄ is a potent mitogen for human peripheral blood lymphocytes and other human cells. By virtue of this particular property RPbAI isoform [a]₄ can in principle be used for all therapeutical applications which are based on the mitogenic stimulation of human cells. Examples are:
- stimulation of intestinal growth
- wound healing
- in vitro activation of lymphocytes
RPbAI isoform [a]₄ is a potent mitogen for animal peripheral blood lymphocytes and other animal cells. By virtue of this particular property RPbAI isoform [a]₄ can in principle be used for all veterinary applications which are based on the mitogenic stimulation of animal cells. Examples are:
- stimulation of intestinal growth
- wound healing
- in vitro activation of lymphocytes
RPbAI isoform [a]₄ specifically recognizes complex animal and human N-glycans. By virtue of this particular carbohydrate-binding specificity RPbAI isoform [a]₄ can in principle be used for all diagnostic applications which are based on the recognition of such N-glycans. For the same reason, RPbAI isoform [a]₄ can also be used for the isolation and characterization of specific human and animal glycoconjugates.

### (ii) Isoform [b]₄ of RPbAI

RPbAI isoform [b]₄ is also mitogen for human peripheral blood lymphocytes and other human cells. By virtue of this particular property RPbAI isoform [b]₄ can in principle be used for all therapeutical applications which are based on the mitogenic stimulation of human cells. Examples are:
- stimulation of intestinal growth
- wound healing
- in vitro activation of lymphocytes
RPbAI isoform [b]₄ is a potent mitogen for animal peripheral blood lymphocytes and other animal cells. By virtue of this particular property RPbAI isoform [b]₄ can in principle be used for all veterinary applications which are based on the mitogenic stimulation of animal cells. Examples are:
- stimulation of intestinal growth
- wound healing
- in vitro activation of lymphocytes
RPbAI isoform [b]₄ specifically recognizes complex animal and human N-glycans. By virtue of this particular carbohydrate-binding specificity RPbAI isoform [b]₄ can in principle be used for all diagnostic applications which are based on the recognition of such N-glycans. For the same reason, RPbAI isoform [b]₄ can also be used for the isolation and characterization of specific human and animal glycoconjugates.

## Claims

1. Method for the isolation of legume lectins, in particular one or more of the tetramers a₄, a₃b₁, a₂b₂, a₃b₁ and b₄ comprising the following steps:
(i) an extraction of legume lectins from raw material,
(ii) a preparation of a partially purified albumin fraction by selective precipitation of non-lectin proteins and polysaccharides
(iii) a purification comprising a chromatography step.

2. Method according to claim 1, **characterized in that** the purification of the fractions of a₄ and b₄ obtained in (iii) are crystallized upon standing in a cold environment, preferably from 0-10°C.

3. Method according to claim 2, **characterized in that** a recrystallization is performed preceded by an additional chromatography step.

4. Method according to any of the claims 1-3, whereby the chromatography steps are ion exchange chromatograph steps.

5. Method according to any of the claims 1-4, whereby the raw material is originating from plants of the leguminoseae family, in particular the tree Robinia pseudoacacia (or black locust), and more in particular the seeds and/or bark thereof.

6. Method according to any of the claims 1-5, whereby the
(i) extraction step further comprises
that the raw material is fractionated for example milled and then extracted in an aqueous medium comprising ascorbid acid, whereafter the pH of the obtained extract is adjusted between 2 and 4, more prefered between 2,5 and 3,5 and most prefered around 3.

7. Method according to any of the claims 1-6, whereby the
(ii) selective precipitation step further comprises centrifugation of the obtained extract, subsequently a decantation of the supernatant, whereby a salt is added, preferably CaCl₂ and whereby the pH is raised between 8 and 10,5 and preferably around 9 such that any co-extracted polysaccharides precipitate,
and further a decantation of the obtained supernatant.

8. Method according to any of the claims 1-7, whereby the obtained supernatant in step (ii) is being purified by ion exchange chromatography, whereby the resulting concentration of the obtained fraction of the isoforms a₄, b₃a₁, b₂a₂, b₁a₃ and/or b₄ is at least 5 mg/ml and preferably 10 mg/ml or more.

9. Purified legume lectins, in particular the isoforms a₄ and/or b₄, obtained in accordance with the method of any of the claims 1-8, for use as a medicament, in particular as a mitogen compound, and more in particular in the treatment of cancer.

10. Purified legume lectins, in particular the isoforms a₄ and/or b₄, obtained in accordance with the method of any of the claims 1-8, for use as a diagnotic compound based on N-glycans.
